# EUROPEAN PATENT APPLICATION

(11) **EP 4 008 332 A1**
(43) Date of publication of application: **08.06.2022**
(21) Application number: 21000279.6
(22) Date of filing: 01.10.2021
(51) Int. Cl.: A61K 31/7072, A61K 31/734, A61K 33/06, A61K 33/08, A61K 36/00, A61P 17/00, A61P 19/00, A61P 21/00, A61P 29/00, A61P 43/00

(54) **PRODUCT FOR PERFORMING A CELLULAR DISINFLAMMATION / REGENERATION AND ITS USE TO ACCELERATE THE REHABILITATION PROCESS**

(30) Priority: 07.12.2020 IT 202000030029
(71) Applicant: Re.Ge.Co. S.r.l., 15029 Solero (AL) (IT)
(72) Inventor: Ronconi, Rita, I-15029 SOLERO (AL) (IT); Massobrio, Luca, I-15029 SOLERO (AL) (IT)
(74) Representative: Garavelli, Paolo

(57) **Abstract**

A product and its use are described to perform a cellular inflammation/regeneration in the rehabilitation process comprising a mixture of sodium alginate in a percentage in weight between 50% and 90% and mineral oxides in a percentage in weight between 10% and 50 %.

## Description

The present invention refers to a product for carrying out cellular inflammation/regeneration and its use to accelerate the rehabilitation process.

In particular, the invention refers to the use of the product to allow a rapid recovery from inflammatory pathologies and traumas of the musculoskeletal system (for example muscle tears, blunt and post-operative traumas, chronic degenerative processes), facilitating the absorption of active ingredients such as antiinflammatories, muscle relaxants.

Treatments are known that use radio-frequency based electro-medical equipment (for example of the type known as CIM200) TECAR Resistive Capacitive Energy Transfer.

It is also known, in the rehabilitation context, to use massage oils composed of medicated oleolites (for example arnica, St. John's Wort) enhanced by essential oils, which due to the high molecular weight are absorbed in a very limited way.

Object of the present invention is providing a product for carrying out cellular inflammation / regeneration and its use to accelerate the rehabilitation process, regenerating tissues and also improving their aesthetic appearance.

The aforementioned and other objects and advantages of the invention, as will emerge from the following description, are achieved with a product for carrying out cellular inflammation / regeneration and its use to accelerate the rehabilitation process such as those described in the independent claims. Preferred embodiments and non-trivial variants of the present invention form the subject of the dependent claims.

It is understood that the attached claims form an integral part of the present description.

It will be immediately obvious that innumerable variations and modifications (for example relating to shape, dimensions, arrangements and parts with equivalent functionality) can be made to what is described without departing from the scope of the invention as appears from the attached claims.

The present invention will be better described by a preferred embodiment, given by way of nonlimiting example.

In a first embodiment, the product for carrying out cellular inflammation/regeneration in the rehabilitation process of the invention comprises a slurry of sodium alginate in a percentage in weight ranging from 50% to 90% and mineral oxides in a percentage in weight ranging from 10 % and 50%.

The invention also includes the use of the aforementioned product and natural essential oils on an anatomical part to be treated for anti-inflammation/cell regeneration in the rehabilitation process.

The invention also describes the use of the product according to the first embodiment, of natural essential oils and electro-medical equipment based on TECAR radio frequency, on an anatomical part to be treated for anti-inflammation/cell regeneration in the rehabilitation process.

In a second embodiment of the invention, the product for carrying out cellular inflammation / regeneration in the rehabilitation process comprises a mixture of calcium sulphate hemihydrate in a percentage in weight between 60% and 90%, water in a percentage in weight between 10% and 40%, essential oils in a percentage in weight between 0% and 5%.

The invention also describes the use of the product according to the second embodiment and of natural essential oils on an anatomical part to be treated for anti-inflammation/cell regeneration in the rehabilitation process.

The method for accelerating the cellular inflammation/regeneration process in the described rehabilitation process comprises the following steps:
- a first step in which a massage of the part to be treated is performed with essential oils and oleolites of natural origin;
- a second step in which sodium alginate is mixed in a percentage in weight between 50% and 90% with mineral oxides, for example magnesium oxide, in a percentage in weight between 10% and 50%;
- a third step in which the mixture of alginate and mineral oxides is applied to the anatomical part to be treated and left on for at least 10 minutes to allow the oleolites to be absorbed by occlusion;

- a fourth step in which the mixture of alginate and mineral oxides is removed;
- a fifth step of use on the anatomical part to be treated of electro-medical equipment based on the radio frequency TECAR, to trigger the self-repair process, acting on tissues prepared in the previous step;
- a sixth step of sealing with oleolites necessary to maintain the optimal state of the treated part;
- a seventh step in which calcium sulphate hemihydrate is mixed in a percentage in weight between 60% and 90%, with water in a percentage in weight between 10% and 40%, essential oils in a percentage in weight between 0% and 5% and various additives (for example Rochelle salt) regulating the setting time and expansion of the mixture in a percentage in weight lower than 1%;
- an eighth step in which, after protecting the area to be treated, a compress is made with the mixture prepared in the seventh step, until drying;
- a ninth step in which the dried dough is removed.

In a second embodiment, the method comprises only the following steps: first, second, third, fourth, sixth.

In a third embodiment, the method comprises only the following steps: first, second, third, fourth, fifth, sixth.

In a fourth embodiment, the method comprises only the following steps: first, sixth, seventh, octave, ninth.

Advantageously, the product to accelerate the cellular inflammation/regeneration process and its use to accelerate the rehabilitation process according to the invention, allow obtaining: an increase in the effectiveness of the single techniques that are merged into a single synergistic treatment, a reduction of functional recovery times, tissue regeneration (for example muscle tear, blunt and post-operative trauma, chronic degenerative processes), restoration of the extracellular matrix, an improvement of the tissue from the aesthetic point of view of scars, cellulite and tissue and adherent relaxation.

## Claims

1. Product for performing a cellular inflammation/regeneration in the rehabilitation process comprising a mixture of sodium alginate in a percentage in weight between 50% and 90% and mineral oxides in a percentage in weight between 10% and 50%.

2. Product for performing a cellular inflammation/regeneration in the rehabilitation process comprising a mixture of calcium sulphate hemihydrate in a percentage in weight between 60% and 90%, water in a percentage in weight between 10% and 40%, essential oils in a percentage in weight between 0% and 5%.

3. Use of the product according to claim 1 and of natural essential oils on an anatomical part to be treated for cellular inflammation/regeneration in the rehabilitation process.

4. Use of the product according to claim 1, of natural essential oils and electro-medical equipment based on TECAR radiofrequency, on an anatomical part to be treated for cellular inflammation/regeneration in the rehabilitation process.

5. Use of the product according to claim 2 and of natural essential oils on an anatomical part to be treated for cellular inflammation/regeneration in the rehabilitation process.
